# EUROPEAN PATENT APPLICATION

(11) **EP 3 401 683 A1**
(43) Date of publication of application: **14.11.2018**
(21) Application number: 17170430.7
(22) Date of filing: 10.05.2017
(51) Int. Cl.: G01N 33/68

(54) **DIAGNOSING METABOLIC DISEASE BY THE USE OF A BIOMARKER**

(71) Applicant: Eberhard Karls Universität Tübingen Medizinische Fakultät, 72074 Tübingen (DE)
(72) Inventor: Lehmann, Rainer, 72074 Tübingen (DE); Häring, Hans-Ulrich, 70597 Stuttgart (DE); Xu, Guowang, Dalian, 116023 (CN); Yin, Peiyuan, Dalian, 116023 (CN); Hoene, Miriam, 72076 Tübingen (DE); Peter, Andreas, 72074 Tübingen (DE)
(74) Representative: Witte, Weller & Partner Patentanwälte mbB

(57) **Abstract**

The present invention relates to a method of diagnosing or aiding in diagnosing whether a subject has a metabolic disease, wherein the method comprises: providing a biological sample from a subject, wherein the subject was previously undergoing metabolic stress; measuring the amount of at least one biomarker for metabolic disease present in the biological sample; comparing the amount of the at least one biomarker in the biological sample with the amount of said at least one biomarker in a healthy reference subject; and identifying a metabolic disease of the subject if the amount of the at least one biomarker present in the biological sample is increased or decreased relative to the amount in the healthy reference subject.

## Description

The present invention relates to the field of diagnosing or aiding in diagnosing whether a subject has a metabolic disease, especially an endocrine disease. The invention more specifically relates to the use of a metabolic marker which can be detected in a biological sample to screen for a metabolic disease.

### FIELD OF THE INVENTION

The invention relates to the field of molecular biology and molecular medicine, in particular the diagnosis of metabolic diseases, including diabetes mellitus.

### BACKGROUND OF THE INVENTION

Interest in metabolic diseases is growing at a considerable rate. A metabolic disease of special interest is diabetes in which patients have elevated blood sugar levels over a prolonged period of time. Symptoms of elevated blood sugar levels include frequent urination, increased thirst, and increased hunger. If left untreated, diabetes can cause many complications.

Diabetes is due to either the pancreas not producing enough insulin or the cells of the body not responding properly to the insulin produced. Diabetes is not a single disease, but an array of diseases which exhibit the common symptoms of glucose intolerance and impairment in glucose utilization. In general, the following three main types of diabetes have been recognized: type I diabetes mellitus, type II diabetes mellitus and gestational diabetes mellitus (GDM).

The WHO defines gestational diabetes mellitus as carbohydrate intolerance, resulting in hyperglycaemia of variable severity with onset or first recognition during pregnancy. GDM generally results in few symptoms; however, it does increase the risk of pre-eclampsia, depression, and requiring a Caesarean section. Babies born to mothers with poorly treated gestational diabetes are at increased risk of being too large, having low blood sugar levels after birth, and jaundice. If untreated, it can also result in a stillbirth. In a long term, children are at higher risk of being overweight and developing type II diabetes.

A number of different methods exists for determining a condition of intolerance for glucose. These are based on blood tests including fasting plasma glucose (FPG) test, HbA1C test, oral glucose tolerance test (oGTT) and random plasma glucose test.

The oGTT is used most frequently. It evaluates how quickly a subject can restore its blood glucose level to a normal level following the ingestion of a large amount of glucose, i.e. it measures a subject's ability to maintain glucose homeostasis. Many variations of the GTT have been devised over the years for various purposes, with different standard dose of glucose, different routes of administration, different intervals and durations of sampling, and various substances measured in addition to blood glucose.

GDM, however, is most readily detected when the carbohydrate metabolic capacity is tested. This is done by stressing the circulation system with a defined glucose load as in the oGTT. The oGTT is a two hour test which determines the blood glucose levels before and two hours after an oral standard dose of glucose is ingested by mouth.

The oGTT has been criticized, however, because many of the variables effecting test results are difficult and laborious to control. For instance: patients must be on a standardized carbohydrate diet at least three days before the test; the test should only be performed on ambulatory patients, therefore a stay in a clinic is often obligatory; three blood samples are necessary, taken before, during and after the oGTT; stress and exercise should be avoided; hormone imbalance can affect validity; various drugs and medications can affect validity; and evaluation should normally be corrected for age.

The greatest disadvantage of the oGTT is that the results are poorly reproducible and this limits its diagnostics usefulness. Therefore, the use of glucose challenge to screen and diagnose GDM is limited by a lack of reproducibility and accuracy of the obtained results, excessive time involved and high costs, late gestational age of testing, nausea and the discomfort of venipuncture. For this reason, the provision of new, innovative methods for diagnosing a metabolic disease is of the outmost importance.

Against this background it is an object underlying the invention to provide a method for diagnosing a metabolic disease, which is sensitive in order to provide data for patient treatment, and in which the disadvantages of the approaches and technologies in the prior art can be avoided or reduced, respectively.

### SUMMARY OF THE INVENTION

This object is met by a method of diagnosing or aiding in diagnosing whether a subject has a metabolic disease, the method comprising:
(a) providing a biological sample from a subject, wherein the subject was previously undergoing metabolic stress;
(b) measuring the amount of at least one biomarker for metabolic disease present in the biological sample;
(c) comparing the amount of the at least one biomarker in the biological sample with the amount of said at least one biomarker in a healthy reference subject; and
(d) identifying the metabolic disease of the subject if the amount of the at least one biomarker present in the biological sample is increased or decreased relative to the amount in the healthy reference subject.

The inventors found out that the amount of at least one biomarker from a biomarker pattern of a healthy subject, which was previously undergoing metabolic stress differs from the amount of the same biomarker in a sick subject which was also undergoing metabolic stress before a biological sample was obtained. In other words, a healthy subject has a different biomarker pattern and therefore a different amount of biomarkers than a subject with a metabolic disease, after both subjects were undergoing metabolic stress.

Thus, the inventors found out that there is a connection between the amount of at least one biomarker of a biomarker pattern from a subject and a metabolic disease of a subject. Moreover the inventors found out that this connection can be properly identified if the subject was undergoing metabolic stress. Therefore, the metabolic stress leads to different amounts of biomarkers in a subject.

According to the invention "metabolic disease" refers to a metabolic disorder which disrupts the normal metabolic process. In other words, a metabolic disease is a disturbance of the internal homeostasis of the body, brought about by an abnormal change in the rate of one or more critical metabolic processes. Therefore, the metabolic disease is characterized by abnormal chemical reactions in the subject's body, which alter the normal metabolic process. It can be the result of an inherited gene abnormality. Because of the disruption of the normal metabolic process an overproduction and/or underproduction of metabolic products, like specific biomarkers, in the subject are the consequence. The metabolic imbalance may result in metabolic diseases. Examples for metabolic diseases are: glucose metabolism disorders, e.g. diabetes mellitus, acid-base imbalance, metabolic brain disease, calcium metabolism disorders, DNA repair-deficiency disorders, hyperlactatemia, iron metabolism disorders, lipid metabolism disorders, inborn error of metabolism, and so on.

The definition of metabolic disease should not be interpreted too broadly. Even infectious diseases, such as those caused by viruses and bacteria exert their clinical effects by altering the internal homeostasis of the body, but these diseases are not primarily metabolic in character.

According to the invention "metabolic stress" refers to any kind of metabolic stimulation. Therefore, metabolic stress is characterized by a disruption in the subject's chemical environment due to the effects of metabolic stimulation. Metabolic stress is not understood in this context as a disease or injury which may also disrupt the subject's chemical environment. Metabolic stimulation can be achieved by the administration of nutrients or physical exercise. The nutrient might be a defined dose of glucose, e.g. similar to the oGTT, and the physical exercise might be any kind of sporting activities.

"Biomarker" means a compound, preferably a metabolite. It belongs to a group of substances which is taking part in a particular metabolic process in a subject and which can be measured in the subject. The biomarker might be an intermediate or a product of a metabolic process. The analysis of biomarkers in this context is also defined as metabolomics or metabolome analysis.

According to the invention "amount" refers to the absolute or relative amount or concentration of a substance, the presence or absence of a substance, the range of amount or concentration of a substance, a minimum and/or maximum amount or concentration of a substance, a mean amount or concentration of a substance, and/or a median amount or concentration of a substance; and, in addition, when analyzing a combination of substances also the ratios of absolute or relative amounts or concentrations of two or more substances with respect to each other may be measured. A substance in this context is preferably a biomarker.

According to the invention "sample" or "biological sample" refers to biological material isolated from the subject. Examples for biological samples are: any suitable biological tissue or fluid such as blood, blood plasma, blood serum, urine, cerebral spinal fluid (CSF), crevicular fluid, salvia or breath condensate.

According to the invention "subject" refers to any human or non-human animal. The term non-human animal includes all vertebrates, e.g. mammals and non-mammals, such as non-human primates, monkeys, rats, mice, sheep, dogs, cows, cats, horses, rabbits, pigs, chicken, amphibians, reptiles, rodents, etc. "Healthy reference subject" means a subject which is on a normal, healthy state.

The inventors found out that a metabolic stimulation caused by metabolic stress leads to a metabolization of substances, small molecules in the body of the subject before they are metabolized to special biomarkers. Thus, the metabolic stress induces a biomarker pattern in the affected subject, which is different to the subject's biomarker pattern without metabolic stress. I.e. different biomarkers and or different amounts of biomarkers are generated before and after the stress. Moreover the biomarker pattern, especially the amount of specific biomarker, of a healthy subject differs from those subjects having a metabolic disease. The amount of the measured biomarker from a sick subject can be higher, lower or equal to the amount of the healthy reference subject. A deviation in the amounts of biomarkers of a healthy subject and a sick subject may be indicative of a particular metabolic disease.

A biomarker may be differently present in healthy and non-healthy subjects by any amount. The different amounts might be in a percentage of at least 10 wt.-%, preferably 15, 20, 25, 30, 40, 50, 60, 70, 80, 90, 100, 200, 300 or 400 wt.-%. A biomarker is preferably differently present by an amount which is statistically significant.

The inventors found out that specific biomarkers can be used to detect, diagnose and monitor a metabolic disease, for example an endocrine disease affecting glucose homeostasis, as well as to guide in the monitoring and selection of the appropriate treatment.

Furthermore, only one biological sample is sufficient to identify a metabolic disease. As a consequence, the subject is only treated once to provide a biological sample.

The method according to the invention offers sufficient accuracy. Thus, the known diagnosing tests, e.g. the known oGTT test, can be replaced.

The inventors have surprisingly realized that with the method according to the invention diagnosing a metabolic disease is more sensitive than the known diagnostic methods in the art. Furthermore the results obtained from the method are reproducible and the method is more reliable than the known diagnostic methods and very fast in obtaining a result.

The availability of an accurate method which can be used for diagnosing or aiding in diagnosing for a metabolic disease provides an important advance in the diagnostic field.

The object underlying the invention is herewith completely met.

In an embodiment of the invention the at least one biomarker for metabolic disease is selected from the group consisting of thyronine, preferably L-thyronine; glycyl-lysine; decadienoylcarnitine, preferably 2-trans-4-cis-decadienoylcarnitine; tryptophanamide; trimethyluric acid, preferably 1,3,9-trimethyluric acid; acetylcarnitine, preferably L-acetylcarnitine; hydroxyadipic acid, preferably 2-hydroxyadipic acid; hydroxybuturic acid, preferably 2-hydroxybuturic acid; acetyl-homoserine, preferably o-acetyl-L-homoserine; hexenedioic acid, preferably 3-hexenedioic acid; serinyl-cystein; isovaleric acid, preferably α-keto-isovaleric acid; myoinositol; glutaconic acid; phosphoric acid; methylhypoxanthine, preferably 1-methylhypoxanthine; hydroxyisovaleric acid, preferably 3-hydroxyisovaleric acid; acetoacetic acid; glutaric acid; aconitic acid; malic acid, preferably L-malic acid; hydroxyvaleric acid, preferably 3-hydroxyvaleric acid and acetyl-homoserine.

The biomarkers described herein were discovered using metabolomic profiling techniques. Such metabolomic profiling techniques are described in more detail in the article "Nontargeted modification-specific metabolomics study based on liquid chromatography- high resolution mass spectrometry" W. Dai et al. Anal. Chem, 2014, 86, 9146 - 9153. The disclosure of this publication in its entirety is hereby incorporated by reference into this application in order to describe more fully said techniques.

The inventors found out, that especially an increase or decrease of one of these listed biomarkers in a biological sample of a subject may be indicative for a metabolic disease, especially an endocrine disease affecting glucose homeostasis. Furthermore, any combination of these biomarkers may be also indicative for a metabolic disease.

A more reliable diagnosing test result can be achieved with the measuring of more than one biomarker, for example two, three, four, five, six, seven, eight, nine, ten or more biomarkers. The biomarker or biomarker combination which is actually measured depend on which metabolic disease is to be diagnosed.

In a further embodiment of the invention the at least one biomarker for metabolic disease is selected from the group of biomarkers listed in table 1:

**Table 1: HR-mass spectrometry of biomarkers. Ion mode = ionization mode (pos = positive mode; neg = negative mode); m/z = mass to charge of the exact mass. unknown = biomarkers where the exact mass and the retention time is known.**

| **No.** | **Ion mode** | **Biomarkers** | **Retention time [min]** | **m/z** |
|---|---|---|---|---|
| 1 | pos | L-Thyronine | 0.656 | 274.1042 |
| 2 | pos | Glycyl-Lysine | 3.518 | 204.1319 |
| 3 | pos | 2-trans,4-cis-Decadienoylcarnitine | 3.546 | 312.2179 |
| 4 | pos | unknown | 2.397 | 204.16 |
| 5 | pos | unknown | 0.842 | 384.1334 |
| 6 | pos | unknown | 0.564 | 223.093 |
| 7 | pos | unknown | 1.956 | 357.1302 |
| 8 | pos | Tryptophanamide | 2.967 | 204.1148 |
| 9 | neg | 1,3,9-Trimethyluric acid | 0.474 | 209.0655 |
| 10 | neg | L-Acetylcarnitine | 3.407 | 202.1072 |
| 11 | neg | unknown | 0.481 | 277.0252 |
| 12 | neg | unknown | 0.4 | 315.0913 |
| 13 | neg | unknown | 1.212 | 225.06 |
| 14 | neg | unknown | 0.4 | 225.06 |
| 15 | neg | 2-Hydroxyadipic acid | 0.74 | 161.0445 |
| 16 | neg | 2-Hydroxybutyric acid | 0.741 | 103.0396 |
| 17 | neg | o-acetyl-L-homoserine | 1.554 | 161.0443 |
| 18 | neg | 3-Hexenedioic acid | 0.426 | 143.0344 |
| 19 | neg | unknown | 1.973 | 355.1129 |
| 20 | neg | unknown | 0.476 | 283.1018 |
| 21 | neg | unknown | 0.435 | 255.0708 |
| 22 | neg | Serinyl-Cysteine | 1.215 | 207.0494 |
| 23 | neg | Alpha-ketoisovaleric acid | 1.06 | 115.0396 |
| 24 | neg | unknown | 0.38 | 215.0197 |
| 25 | neg | unknown | 0.4 | 215.0313 |
| 26 | neg | unknown | 2.275 | 217.0257 |
| 27 | neg | Myo-inositol | 1.983 | 179.055 |
| 28 | neg | unknown | 2.399 | 161.0444 |
| 29 | neg | unknown | 3.414 | 225.0598 |
| 30 | neg | unknown | 2.148 | 143.034 |
| 31 | neg | Glutaconic acid | 0.447 | 129.0185 |
| 32 | neg | Phosphoric acid | 0.362 | 96.96889 |
| 33 | neg | 1-Methylhypoxanthine | 0.429 | 149.0446 |
| 34 | neg | 3-Hydroxyisovaleric acid | 1.902 | 117.0552 |
| 35 | neg | Acetoacetic acid | 1.233 | 101.0239 |
| 36 | neg | Glutaric acid | 1.527 | 131.0341 |
| 37 | neg | Aconitic acid | 0.635 | 173.0079 |
| 38 | neg | L-Malic acid | 0.427 | 133.0136 |
| 39 | neg | 3-Hydroxyvaleric acid | 1.149 | 117.0551 |
| 40 | neg | unknown | 4.007 | 615.3102 |

A biomarker pattern of forty biomarkers has been identified by the inventors which, alone or in various combinations, ensure a high degree of accuracy in identifying a metabolic disease, especially an endocrine diseases affecting glucose homeostasis.

An increase or decrease of at least one biomarker listed in table 1 in a biological sample obtained from a subject can be used for identifying a metabolic disease of the subject. The respective biomarkers or a combination of these biomarkers are specific for a metabolic disease. For example the inventors found out, that measuring different amounts of all forty listed biomarkers, compared to the amount of a healthy reference subject, identify a metabolic disease, especially an endocrine disease affecting glucose homeostasis, by a very high sensitivity, specificity and accuracy.

In another embodiment of the invention the at least one biomarker for metabolic disease is the combination of biomarkers no. 5, no. 10, no. 11 and no. 35 listed in table 1.

The inventors were able to find out that measuring the amount of the combination of these four biomarkers and comparing the amount of these biomarkers to the amount of the same biomarkers of a healthy reference subject may be especially suitable for diagnosing a metabolic disease, more especially an endocrine disease affecting glucose homeostasis. For example, with the preferred four biomarkers an area under the receiver-operating-characteristics-curve (AUC) of 0.917 can be reached in diagnosing GDM.

In a further embodiment of the invention the at least one biomarker is the combination of biomarkers no. 1, no. 5, no. 9, no. 10, no. 11, no. 15, no. 16, no. 17, no. 18, no. 22, no.34, no. 36, and no. 40, more preferably the combination of biomarkers no. 9, no. 10, no. 15, no. 16, no. 17, no. 18, no. 34 and no. 36 listed in table 1.

The inventors were able to find out that measuring the amount of the combination of these eight or thirteen biomarkers and comparing the amount of these biomarkers to the amount of the same biomarkers of a healthy reference subject may be especially suitable for diagnosing a metabolic disease, especially an endocrine disease affecting glucose homeostasis. With the preferred thirteen biomarkers an AUC of 0.972 can be reached in diagnosing GDM. Also, with the preferred eight biomarkers an AUC of 0.886 can be reached in diagnosing GDM. The measurement and comparison of both biomarkers' combinations lead to an excellent accuracy in diagnosing a metabolic disease.

In another embodiment of the invention the number of measured biomarkers is two or more, three or more, four or more, five or more, six or more, seven or more, eight or more, nine or more, ten or more, thirteen or more, fifteen or more, twenty or more, thirty or more or forty or more.

The more biomarkers in combination are measured and compared to the healthy reference subject the more reliable the test result might be for diagnosing a metabolic disease. Additional markers can be used to improve the diagnosis or aid in diagnosing, including any combination of two, three, four, five, six, seven, eight, nine, ten, thirteen, fifteen, twenty, thirty, forty, or more biomarkers described herein. Which combination of measured biomarkers is useful depends on which metabolic disease is to be diagnosed.

In a further embodiment of the invention the metabolic stress which the subject was previously undergoing is caused by a physical exercise.

The physical exercise of a subject will result in a stimulation of the metabolism of a subject. Thus, small molecules are metabolized into specific biomarkers, wherein their amount depends on the healthy or unhealthy state of the subject.

In another embodiment of the invention the metabolic stress which the subject was previously undergoing is caused by an administration of a dose of glucose, wherein the dose is preferably 100 g oral glucose, 75 g oral glucose or 50 g oral glucose.

The administration of glucose may induce glycolysis, carbon fixation, glycogenesis, glycogenolysis, gluconeogenesis or the pentose phosphate pathway, and thus triggering the metabolization of specific biomarkers. This metabolization can be analyzed by measuring the amount of specific biomarkers. Therefore, a measured increase, decrease or absence of specific biomarkers concludes a metabolic disease of a subject. This can be sensitively and specifically reached.

The administration of glucose described herein can be via any of the accepted modes of administration. These methods include non-invasive modes like peroral, topical, transmucosal and inhalation modes or invasive modes. Therefore glucose may be dissolved or suspended in a hydrophilic or partially hydrophilic solvent.

In a further embodiment of the invention the measuring in step (b) is performed by using one or more techniques selected from the group consisting of gas chromatography, liquid chromatography, mass spectrometry, immunoassay, ELISA, enzymatic or biochemical reactions and NMR, or combinations of two or more of these methods.

The listed techniques have the advantage that the measurement of the amount of the at least one biomarker in a biological sample can be reached in a high sensitivity and in a high specificity. The measuring of the amount of the at least one biomarker can also reached very fast. Depending on which biomarker is measured or which kind of biological sample is used different measurement technique may be preferred.

Furthermore, this embodiment has the advantage that different kinds of measurement techniques can be used. This can be advantageously for some diagnostic laboratories where some of the listed techniques are already used. Therefore, no specific or new means for measuring the biomarker have to be purchased for carrying out the method according to the invention.

The measured amount of the at least one biomarker in a biological sample can be compared directly to the measured amount of the same biomarker of a healthy reference subject, such as comparing the concentration of the at least one biomarker present in the biological sample to the concentration of the same biomarker in a corresponding healthy reference sample. The both amounts are preferably measured with the same technique to avoid discrepancies caused by the use of different techniques. E.g. a mass-spectrometry may be used to determine the presence or absence of a measured biomarker in a sample, wherein an immunoassay of a sample may be used to obtain an absolute or relative amount or concentration of a measured biomarker in a sample.

In another embodiment of the invention the metabolic disease is selected from the group consisting of diabetes mellitus, especially gestational diabetes mellitus, type 2 diabetes mellitus, impaired glucose tolerance, metabolic syndrome, pre-diabetes and another endocrine diseases affecting glucose homeostasis.

This embodiment has the advantage, that the listed diseases can be diagnosed by the method according to the invention. The known methods in the art for diagnosing these metabolic diseases are much more uncomfortable, more expensive and more time consuming, than the method according to the invention. Furthermore, the results obtained from the methods known in the art are less accurate.

All the listed metabolic diseases are endocrine diseases affecting glucose homeostasis. Endocrine disease is a disorder of the endocrine system. Glucose homeostasis is the balance of glucoregulatory hormones like insulin and glucagon to maintain the blood glucose level in a normal range.

For example, the invention provides a method of diagnosing or aiding in diagnosing a metabolic disease, especially a metabolic disease which occurs with an abnormal glucose metabolism, e.g. diabetes mellitus. The method is particularly useful for identifying susceptibility to gestational diabetes mellitus (GDM) during early pregnancy, including during the first trimester of pregnancy as well as later in pregnancy and postpartum. The invention also provides a method of detecting diabetes in a subject.

In a further embodiment of the invention the biological sample is selected from the group consisting of sputum, blood, plasma, serum, lactation products, amniotic fluids, saliva, semen, cerebrospinal fluid, bronchial aspirate, perspiration, mucus, liquefied stool sample, synovial fluid, lymphatic fluid, tears and tracheal aspirate, preferably urine.

This embodiment has the advantage, that each biological sample of the listed group of biological samples can be obtained in a routine visit at a doctors office or the subject can obtain the biological sample by its own. Especially the biological sample, which is obtained noninvasive, has the major advantage that the withdrawing is painless and the sample is easily to collect.

For example a urine sample can be collected at a routine visit. The collection therefore does not involve a highly trained, expensive nurse or phlebotomist, as may be required for other samples. This additional advantage will allow metabolic disease diagnosing with limited resources.

Another subject matter of the present invention relates to a use of a biomarker pattern consisting of at least one biomarker of a biological sample from a subject, wherein the subject was previously undergoing metabolic stress for diagnosing or aiding in diagnosing whether a subject has a metabolic disease.

In another embodiment of the use according to invention the at least one biomarker for metabolic disease is selected from the biomarkers listed in table 1.

The features, characteristics, embodiments and advantages of the method according to the invention apply to the use according to the invention correspondingly.

A further subject matter of the present invention relates to a test kit for diagnosing or aiding in diagnosing whether a subject has a metabolic disease, said test kit comprising: means for determining the amounts of at least one biomarker for a metabolic disease present in a biological sample from a subject, wherein the subject was undergoing metabolic stress, wherein the determined biomarker is selected from the group of biomarkers described herein.

The features, characteristics, embodiments and advantages of the method according to the invention apply to the test kit according to the invention correspondingly.

This embodiment has the advantages that diagnosing of a metabolic disease of a subject can be performed very fast in only one device. The test kit may be arranged in a way that the biological sample just has to be inserted into the test kit, while the method according to the invention may be carried out fully automatically. Therefore, an unskilled person may be able to use the test kit.

The test kit may include means which are necessary for determining the amounts of at least one biomarker for metabolic disease. This might be chromatographic means, spectrometric means, means for enzymatic or biochemical reactions, etc., as well as means for stabilizing the biological sample. The test kit might not be limited in just one technical means for determining the amount of at least one biomarker. For example the test kit might have means for performing a HPLC (high performance liquid chromatography) or UHPLC (ultra-high performance liquid chromatography) coupled to a mass spectrometry or coupled to an immunoassay.

The test kit may also include reagents which might be necessary for performing the method according to the invention. The reagents may be provided in any suitable form. The reagents might be stabilization reagents, buffer reagents, solvents etc.

In another embodiment of the kit according to the invention the means for determining the amount of the at least one biomarker comprise an immunoassay and/or a mass-spectrometer.

This embodiment has the advantages that determining the amount of at least one biomarker can be realized with high precision and sensitivity.

The embodiment which comprises mass-spectrometer means for determining the amount of the at least one biomarker has the advantages that this technique is able to measure a huge amount of biomarkers in a sample; in other words, with this technique a user might be able to measure the amount from lots of different biomarkers in just one run. Furthermore, mass spectrometry has the advantage that the detection of biomarkers can be realized from micro liter quantities of the biological sample.

The embodiment which comprises immunoassay means for determining the amount of the at least one biomarker has the advantage that this technique is quite cheep in building, but high sensitive in measuring the amount of at least one biomarker.

In this embodiment the immunoassay might be an ELISA. Therefore the test kit may comprise separate compartments, tubes, valves and the like. The test kit might also include reagents which are necessary for performing an immunoassay, like antibodies, buffers, blocking agents, detection reagents and the like. These reagents are well known in the art.

It shall be understood that the features mentioned above and those to be mentioned in the following cannot only be used in the combination indicated in each case but also in other combinations or in isolated positions without departing from the scope of the present invention.

The invention is now explained in more details by means of embodiments which result in further characteristics, features and advantages. The embodiments are purely illustrative and do not restrict the scope of the invention. Features which are described in connection with a specific embodiment are isolated features of the invention in its general form and are not only features in the specific technical context.

Reference is made to the enclosed figures which show the following:
- Fig. 1: shows a heatmap of 50 biomarkers of a comparison of 15 pregnant women without and 15 women with GDM and a comparison of both women groups before and after undergoing metabolic stress;
- Fig. 2: shows two ROC curves resulting from a combination of eight and thir-teen biomarkers; and
- Fig. 3: shows a ROC curve resulting from a combination of four biomarkers.

### EXAMPLES

The following examples are presented to illustrate the present invention and to assist one of ordinary skill in making and using the same. The examples are not intended in any way to otherwise limit the scope of the invention.

The following examples demonstrate that a post-oGTT spot urine sample can be used to identify pregnant women who have developed gestational diabetes mellitus (GDM) and those who will not develop GDM.

The post-oGTT spot urine sample is defined as the first urine sample of the subject after undergoing metabolic stress by taking a defined dose of glucose. Therefore the urine sample is collected at least two hours after the subject took the defined dose of glucose but not earlier. Urine samples, which were obtained during the two hours after taking the defined dose of glucose, are not post-oGTT spot urine samples according to the invention.

### 1. Material and methods

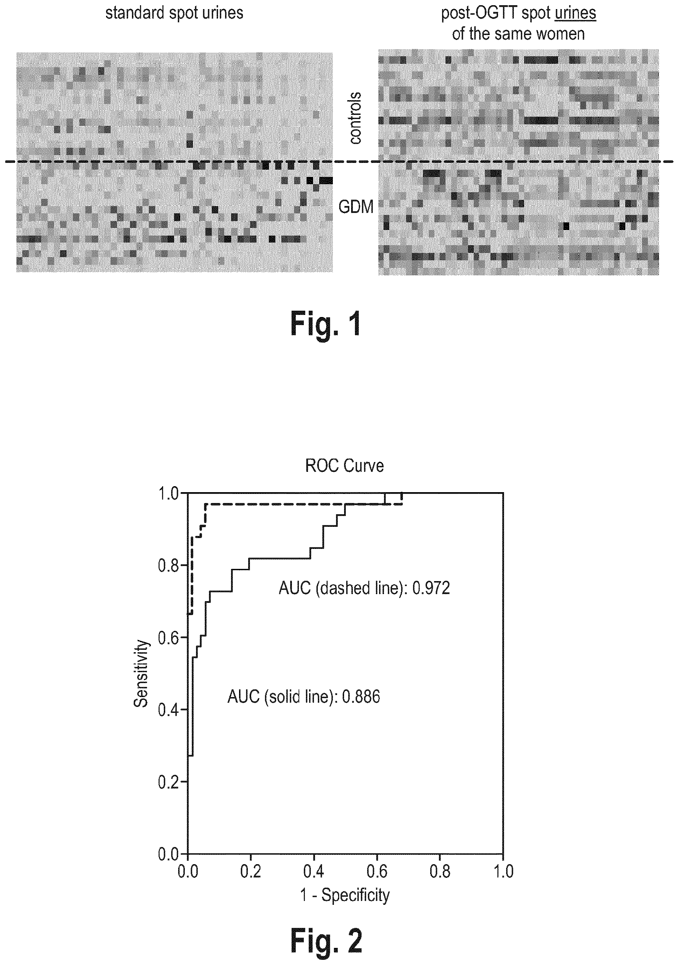

The biomarkers listed in table 1 and the biomarkers shown in the heatmap in Fig. 1 were identified and classified by liquid chromatography-mass spectrometry (LC-MS). All LC-MC analyses were performed with an Acquity UPLC system (Waters, MA, USA) coupled to a LTQ-Oribitrap XL (Thermo Fisher Scientific, Waltham, MA, USA). Chromatographic separation was performed using a BEH T3 column (10 cm × 2.1 mm, 1.7 µm, Waters. Mobile phases A and B were 0.1 % formic acid solution and acetonitrile, respectively. The linear gradient program was as follows: from start to 5 min, phase B was linearly increased from 2% to 5%, then increased to 30% over the next 10 min, and then linearly increased to 98% during the next 6 min and held at 98% for 6 min. The flow rate was 0.28 mL/min. The injection volume for the LC-MS analysis with the ISCID voltage (5-45 V) was 10 µL.

### 2. Screening to elucidate a diagnostic GDM-biomarker in postOGTT spot urine

The biomarker screening has been performed on urine. Urine has particular advantages over blood tests because it is readily available, collectable without discomfort to the patient, and does not require expensively skilled personnel for collection. But other body fluids can also be sampled for biomarker analysis.

Standard spot urine and postOGTT spot urine was collected from 30 pregnant women from 24 - 27 weeks of gestation. Gestational diabetes was determined by standard clinical screening at 24-27 weeks of gestation. The results of the standard clinical screening separates the pregnant women into two groups: 15 normal pregnant women with a very high insulin sensitivity index (ISI ≥ 16) and those 15 pregnant women with GDM and a very low insulin sensitivity index (ISI ≥ 6). The ISI is an index which evaluates whole body physiolgical insulin sensitivity from the data obtained by an oral glucose tolerance test.

First a standard spot urine sample (second morning urine after a 12 h fasting period) was collected from each of the 30 women and was analyzed by ISCID-UPLC-mass spectrometry. Therefore the amounts of 50 different biomarkers were measured. The metabolomic profiling techniques are described in more detail in the article "Nontargeted modification-specific metabolomics study based on liquid chromatography-high resolution mass spectrometry" W. Dai et al. Anal. Chem, 2014, 86, 9146 - 9153. The disclosure of this publication in their entireties is hereby incorporated by reference into this application.

Afterwards the 30 women undergo metabolic stress. For this purpose a 75 g oral glucose challenge test was performed. Therefore the women drank a glucose water mixture of 75 g glucose and 300 mL water. Afterwards the postOGTT spot urine samples were collected from each of the 30 women and than analyzed by ISCID-UPLC-mass spectrometry. Thus, the amounts of the same 50 different biomarkers, which were analyzed from the spot urine samples (postOGTT spot urine samples and standard spot urine samples), were measured. The amounts of the screened biomarkers were analyzed by mass spectrometry.

### Results:

The results of the mass spectrometry of the screening are shown in the heatmaps in Fig. 1. The first heatmap, on the left side, shows 50 biomarkers from 30 different samples, wherein the samples are standard spot urine. 15 samples of the 30 samples are obtained from women without GDM (controls). The other 15 samples are obtained from women with GDM. The second heatmap, on the right side, shows 50 biomarkers from 30 different samples, wherein the samples are postOGTT spot urine from the same women shown in the first heatmap.

Each biomarker is represented by a colored square. The different amounts of the biomarkers are visualized by different colors. The higher (black) or lower (grey) the amount of a biomarker, in comparison to the mean values of the healthy controls, the darker the square of the biomarker.

Each horizontal line from the first as well as from the second heatmap shows the amounts of 50 different biomarkers from one subject. The horizontal lines from both heatmaps, which are on the same high, can be compared to each other, because the samples are from the same subjects.

Each vertical line from the first as well as from the second heatmap shows the amounts of one biomarker from 30 different subjects.

The comparison of both heatmaps points out huge differences. First, the biomarker pattern of a subject, especially the amount of the different biomarkers is different before and after a subject is undergoing metabolic stress. Second, the biomarker pattern, especially the amount of the different biomarkers is different between the group of healthy women and those with GDM.

The comparison shows a connection between the different amounts of biomarkers in a biomarker pattern of a subject before and after metabolic stress and with and without metabolic disease like GDM. Furthermore, the heatmaps pointed out that specific biomarkers have different amounts in the samples of sick and healthy subjects wherein both subject groups were undergoing metabolic stress. These differences in the amount of biomarkers can not be noticed in the sample of sick and healthy subjects which where not undergoing metabolic stress. It can be seen that the metabolic stress leads to different amounts of biomarkers in a sample obtaining from a subject, which was undergoing metabolic stress in comparison to a subject which was not undergoing metabolic stress.

Therefore the inventors found out, that more reliable results of diagnosing a metabolic disease with biomarkers can be obtained, if the subject was previously undergoing metabolic stress.

### 3. Validation of diagnostic GDM biomarker pattern

After the screening of the biomarkers, the inventors found out in a validation that 40 biomarkers of the initial 50 biomarkers are most sensitive in diagnosing GDM. Those biomarkers are listed in table 1. The biomarkers listed in table 1 are based on the results of the screening (n=30) and the validation analyses (n=105).

The validation of the 40 biomarkers was performed as described above, but with 105 subjects, wherein 33 women were suffering from GDM and wherein 72 women were not.

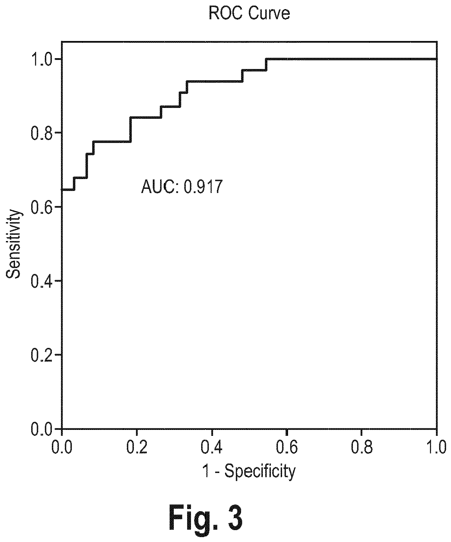

In this context the inventors found out that less than 40 specific biomarkers can be used to diagnose metabolic disease. The number of these specific biomarkers depends on the biomarker itself. Therefore further statistical scrutiny of the biomarkers was made. These results are shown in Fig. 2 and Fig. 3. Three analysis revealed the AUC area under the ROC curve using a combination of four, eight and thirteen biomarkers.

Four, eight and thirteen biomarkers have been identified which, in various combinations, have a high degree of accuracy in diagnosing GDM. In this validation, the postOGTT urinary analysis results in an AUC of 0.917 for four biomarkers, an AUC of 0.886 for eight biomarkers and an AUC of 0.972 for thirteen biomarkers.

The AUC described herein was discovered using the probability values calculated by binary logistic regression. Such binary logistic regression calculation is described in more detail in the article "Discovery of serum biomarkers for pancreatic adenocarcinoma using proteomic analysis." A. Xue et al. BrJCancer 2010, 103, 391 - 400. The disclosure of this publication in its entirety is hereby incorporated by reference into this application in order to describe more fully said calculation.

The four biomarkers resulting in an AUC 0.917 shown in Fig. 3 are: acetoacetic acid, acetylcarnitine, m/z 384.1334, m/z 277.0252; also listed in table 1 as number 5, 10, 11 and 35.

The eight biomarkers resulting in an AUC 0.886 shown as the solid line in Fig. 2 are: trimethyluric acid, acetylcarnitine, hydroxyadipic acid, hydroxybutyric acid, acetyl-homoserine, hexenedioic acid, hydroxyisovaleric acid, glutaric acid; also listed in table 1 as number 9, 10, 15, 16, 17, 18, 34 and no. 36.

The thirteen biomarkers resulting in an AUC 0.972 shown as the dashed line in Fig. 2 are: trimethyluric acid, acetylcarnitine, hydroxyadipic acid, hydroxybutyric acid, acetyl-homoserine, hexenedioic acid, hydroxyisovaleric acid, glutaric acid, thyronine, serinyl-cysteine, m/z 384.1334, m/z 615.3102, m/z 277.0252; also listed in table 1 as number 1, 5, 9, 10, 11, 15, 16, 17, 18, 22, 34, 36, and 40.

## Claims

1. A method of diagnosing or aiding in diagnosing whether a subject has a metabolic disease, the method comprising:
(a) providing a biological sample from a subject, wherein the subject was previously undergoing metabolic stress;
(b) measuring the amount of at least one biomarker for metabolic disease present in the biological sample;
(c) comparing the amount of the at least one biomarker in the biological sample with the amount of said at least one biomarker in a healthy reference subject; and
(d) identifying a metabolic disease of the subject if the amount of the at least one biomarker present in the biological sample is increased or decreased relative to the amount in the healthy reference subject.

2. The method of claim 1, wherein the at least one biomarker for metabolic disease is selected from the group consisting of: thyronine, preferably L-thyronine; glycyl-lysine; decadienoylcarnitine, preferably 2-trans-4-cis-decadienoylcarnitine; tryptophanamide; trimethyluric acid, preferably 1,3,9-trimethyluric acid; acetylcarnitine, preferably L-acetylcarnitine; hydroxyadipic acid, preferably 2-hydroxyadipic acid; hydroxybuturic acid, preferably 2-hydroxybuturic acid; acetyl-homoserine, preferably o-acetyl-L-homoserine; hexenedioic acid, preferably 3-hexenedioic acid; serinyl-cystein; isovaleric acid, preferably α-keto-isovaleric acid; myoinositol; glutaconic acid; phosphoric acid; methylhypoxanthine, preferably 1-methylhypoxanthine; hydroxyisovaleric acid, preferably 3-hydroxyisovaleric acid; acetoacetic acid; glutaric acid; aconitic acid; malic acid, preferably L-malic acid; hydroxyvaleric acid, preferably 3-hydroxyvaleric acid and acetyl-homoserine.

3. The method of any of claims 1 or 2, wherein the at least one biomarker for metabolic disease is selected from the group of biomarkers listed in table 1.

4. The method of claim 3, wherein the at least one biomarker for metabolic disease is the combination of biomarkers no. 5, no. 10, no. 11 and no. 35 listed in table 1.

5. The method of claim 3, wherein the at least one biomarker for metabolic disease is the combination of biomarkers no. 1, no. 5, no. 9, no. 10, no. 11, no. 15, no. 16, no. 17, no. 18, no. 22, no.34, no. 36, and no. 40, more preferably the combination of biomarkers no. 9, no. 10, no. 15, no. 16, no. 17, no. 18, no. 34 and no. 36, listed in table 1.

6. The method of any of the preceding claims, wherein the number of measured biomarkers is two or more, three or more, four or more, five or more, six or more, seven or more, eight or more, nine or more, ten or more, thirteen or more, fifteen or more, twenty or more, thirty or more or forty or more.

7. The method of any of the preceding claims, wherein the metabolic stress which the subject was previously undergoing is caused by a physical exercise.

8. The method of any of the preceding claims, wherein the metabolic stress which the subject was previously undergoing is caused by an administration of a dose of glucose, wherein the dose is preferably 100 g oral glucose, 75 g oral glucose or 50 g oral glucose.

9. The method of any of the preceding claims, wherein the measuring in step (b) is performed by using one or more techniques selected from the group consisting of gas chromatography, liquid chromatography, mass spectrometry, immunoassay, ELISA, enzymatic or biochemical reactions and NMR, or combinations of two or more of these methods.

10. The method of any of the preceding claims, wherein the metabolic disease is selected from the group consisting of diabetes mellitus, especially gestational diabetes mellitus, type 2 diabetes mellitus, impaired glucose tolerance, metabolic syndrome, pre-diabetes and another endocrine diseases affecting glucose homeostasis.

11. The method of any of the preceding claims, wherein the biological sample is selected from the group consisting of sputum, blood, plasma, serum, lactation products, amniotic fluids, saliva, semen, cerebrospinal fluid, bronchial aspirate, perspiration, mucus, liquefied stool sample, synovial fluid, lymphatic fluid, tears and tracheal aspirate, preferably urine.

12. Use of a biomarker pattern consisting of at least one biomarker of a biological sample from a subject, wherein the subject was previously undergoing metabolic stress for diagnosing or aiding in diagnosing whether a subject has a metabolic disease.

13. The use of claim 11, wherein the at least one biomarker is selected from the biomarkers listed in table 1.

14. Test kit for diagnosing or aiding in diagnosing whether a subject has a metabolic disease, said test kit comprising: means for determining the amounts of at least one biomarker for a metabolic disease present in a biological sample from a subject, wherein the subject was undergoing metabolic stress, wherein the determined biomarker is selected from the group of biomarkers listed in any of claim 2 to 5.

15. The test kit of claim 14, wherein the means for determining the amount of the at least one biomarker are configured for performing an immunoassay and/or a mass-spectrometric-based kit.
